Europäisches Patentamt

European Patent Office

Office européen des brevets

(11) Publication number: **0 068 796**
**B1**

(12) **EUROPEAN PATENT SPECIFICATION**

(45) Date of publication of patent specification: 17.09.86

(21) Application number: 82303246.1

(22) Date of filing: 22.06.82

(51) Int. Cl.⁴: **C 01 B 35/10, C 01 B 33/28, B 01 J 29/28**

(54) Method for manufacture of AMS-IB crystalline borosilicate molecular sieve.

(30) Priority: 30.06.81 US 279207
08.06.82 US 386285

(43) Date of publication of application:
05.01.83 Bulletin 83/01

(45) Publication of the grant of the patent:
17.09.86 Bulletin 86/38

(84) Designated Contracting States:
AT BE CH DE FR GB IT LI LU NL SE

(56) References cited:
EP-A-0 000 669
EP-A-0 011 362
DE-A-2 830 787
GB-A-2 024 790
US-A-4 139 600
US-A-4 151 189
US-A-4 268 420
US-A-4 269 813
US-A-4 285 919

(73) Proprietor: **AMOCO CORPORATION**
**200 East Randolph Drive**
**Chicago Illinois 60601 (US)**

(72) Inventor: **Haddad, Muin Shawki**
**5 S 408 Glenoban Drive**
**Naperville Illinois 60540 (US)**

(74) Representative: **Ritter, Stephen David et al**
**Mathys & Squire 10 Fleet Street**
**London EC4Y 1AY (GB)**

Courier Press, Leamington Spa, England.

Description

Background of the Invention

This invention relates to a new method to manufacture molecular sieves and more particularly to a new method to manufacture crystalline borosilicate AMS-1B molecular sieve and to a product made from that method.

Zeolitic materials, both natural and synthetic, are known to have catalytic capabilities for many hydrocarbon processes. Zeolitic materials typically are ordered porous crystalline aluminosilicates having a definite structure with cavities interconnected by channels. The cavities and channels throughout the crystalline material generally are uniform in size allowing selective separation of hydrocarbons. Consequently, these materials in many instances are known in the art as "molecular sieves" and are used, in addition to selective adsorptive processes, for certain catalytic properties. The catalytic properties of these materials are affected to some extent by the size of the molecules which selectively penetrate the crystal structure, presumably to contact active catalytic sites within the ordered structure of these materials.

Generally, the term "molecular sieve" includes a wide variety of both natural and synthetic positive-ion-containing crystalline zeolite materials. They generally are characterized as crystalline alumino-silicates which comprise networks of $SiO_4$ and $AlO_4$ tetrahedra in which silicon and aluminum atoms are crosslinked by sharing of oxygen atoms. The negative framework charge resulting from substitution of an aluminum atom for a silicon atom is balanced by positive ions, for example, alkali-metal or alkaline-earth-metal cations, ammonium ions, or hydrogen ions.

Boron is not considered a replacement for aluminum or silicon in a zeolitic composition. However, recently a new crystalline borosilicate molecular sieve AMS-1B was disclosed in U.S. Patent 4,268,420 and 4,269,813. According to these patents AMS-1B can be synthesized by crystallizing a source of an oxide of silicon, an oxide of boron, an oxide of sodium and an organic template compound such as a tetra-n-propylammonium salt. In order to form a catalytically-active species of AMS-1B, sodium ion typically is removed by one or more exchanges with ammonium ion followed by calcination. Other methods to produce borosilicate molecular sieves include using a combination of sodium hydroxide and aqueous ammonia together with an organic template as disclosed in U.S. Patent 4,285,919, and using high concentrations of amine such as hexamethylenediamine as described in German Patent Application 28 30 787. British Patent Application 2,024,790 discloses formation of a borosilicate using ethylene diamine with sodium hydroxide. Aluminosilicates have been prepared with low sodium content using diamines containing four or more carbon atoms as described in European Published Patent Applications 669 and 11 362. U.S. Patents 4,139,600 and 4,151,189 describe methods to produce aluminosilicate sieves containing low sodium using diamines or $C_2$—$C_5$ alkyl amines. AMS-1B crystalline borosilicates are generally produced in the prior art processes under crystallization conditions utilising a relatively high molar ratio of water to silica. It has now been found that by reducing the proportion of water, products may be formed which when used as catalyst supports provide supported catalysts having increased activity compared to conventionally prepared material.

A method to produce AMS-1B crystalline borosilicate molecular sieve which is low in sodium would be desirable in that an exchange procedure to remove sodium would be unnecessary. Also a method to produce AMS-1B crystalline borosilicate having a higher boron content than usually prepared by conventional techniques would be very advantageous. Further, a method to produce AMS-1B crystalline borosilicate without use of added alkali or ammonium hydroxides would be desirable. In addition a product formed from such method which shows increased activity over conventionally-prepared material would be most advantageous.

Summary of the Invention

This invention is a method to prepare AMS-1B crystalline borosilicate molecular sieve comprising reacting under crystallization conditions, in substantial absence of a metal or ammonium hydroxide, an aqueous mixture containing an oxide of silicon, an oxide of boron, an alkylammonium cation or a precursor of an alkylammonium cation, and ethylenediamine, and the product formed from such method.

Brief Description of the Invention

Conventionally, AMS-1B borosilicate molecular sieve is prepared by crystallizing an aqueous mixture of an oxide of boron, an oxide of silicon, and an organic template compound in the presence of an alkali metal hydroxide, usually sodium hydroxide. When such a mixture is crystallized, the resulting AMS-1B molecular sieve contains alkali metal, usually sodium, ions to balance the negative framework charge caused by substitution of a boron atom for silicon in the crystalline sieve structure. However, when used for catalytic purposes, presence of sodium ion usually is detrimental. Typically, before a catalytic composition is made, the hydrogen form of AMS-1B is prepared by exchange with ammonium ion followed by drying and calcination. This invention is a method of directly crystallizing AMS-1B molecular sieve having a low sodium content which uses less of expensive alkylammonium template compound than used in conventional preparations.

According to the present invention there is provided a method of preparing AMS-1B crystalline borosilicate molecular sieve comprising reacting under crystallization conditions, in substantial absence of a metal or ammonium hydroxide, an aqueous mixture containing an oxide of silicon, an oxide of boron, ethylenediamine and, optionally, an alkylammonium cation or precursor of an alkylammonium cation wherein the molar ratio of silica to oxide of boron is from 2 to 400 and the molar ratio of ethylene diamine to oxide of silicon is above 0.05, characterised in that the molar ratio of water to silica is from 10 to 15.

Typically, the mole ratios of the various reactants can be varied to produce the crystalline borosilicates of this invention. Preferably, the molar ratio of initial reactant concentration of silica to oxide of boron ranges from 4 to 150 and most preferably from 5 to 80. The molar ratio of ethylene diamine to silicon oxide used in the preparation of AMS-1B crystalline borosilicate according to this invention should be above 0.05, typically below 5, preferably 0.1 to 1.0, and most preferably 0.2 to 0.5. The molar ratio of alkylammonium template compound or precursor to silicon oxide useful in the preparation of this invention can range from 0 to 1 or above, typically above 0.005, preferably 0.01 to about 0.1, and most preferably from about 0.02 to about 0.05.

It has been found that AMS-1B crystalline borosilicate molecular sieve formed using the method of this invention in which such sieve is formed in a mixture containing a low water to silica ratio exhibits surprisingly high catalytic activity in hydrocarbon conversion such as in converting ethylbenzene. AMS-1B crystalline borosilicate compositions showing exceptional conversion activity can be prepared by crystallizing a mixture of an oxide of silicon, an oxide of boron, an alkylammonium compound and ethylenediamine such that the initial reactant molar ratios of water to silica range from 10 to 15. In addition, preferable molar ratios for initial reactant silica to oxide of boron range from about 4 to about 150, more preferably about 5 to about 80 and most preferably about 5 to about 20. The molar ratio of ethylenediamine to silicon oxide used in the preparation of AMS-1B crystalline borosilicate according to this invention should be above about 0.05, typically below about 5, preferably about 0.1 to about 1.0, and most preferably about 0.2 to about 0.5. The molar ratio of alkylammonium template compound or precursor to silicon oxide useful in the preparation of this invention can range from 0 to about 1 or above typically above about 0.005, preferably about 0.01 to about 0.1, and most preferably about 0.02 to about 0.05.

It is noted that the preferable amount of alkylammonium template compound used in the preparation of this invention is substantially less than that required to produce AMS-1B conventionally using an alkali metal cation base. The decrease in use of such alkylammonium compound substantially lowers the cost of preparation.

The amount of alkylammonium template used in preparations of this invention generally is in inverse proportion to the amount of ethylenediamine used. If no alkylammonium compound is employed, preparations using ethylenediamine in a molar ratio to silica of above about 1 usually form highly crystalline borosilicate molecular sieves. At molar ratios below about 1 partially crystalline material is formed and at molar ratios below about 0.5 amorphous product is obtained. However, if an alkylammonium compound is included in a preparation using ethylenediamine in a molar ratio to silica less than about 1, crystalline AMS-1B borosilicate is formed. As the proportion of ethylenediamine is decreased, generally the proportion of alkylammonium compound may be increased. Nevertheless, in any peparation of this invention no added hydroxide, such as in the form of an alkali or alkaline earth metal hydroxide or ammonium hydroxide, is used, although in substantial amounts may be present as impurities in starting reagents.

By regulation of the quantity of boron oxide (represented as $B_2O_3$) in the reaction mixture, it is possible to vary the $SiO_2/B_2O_3$ (silica/boria) molar ratio in the final product, although in many instances an excess of boron oxide is used in a preparation.

AMS-1B crystalline borosilicate molecular sieve generally can be characterized by the x-ray pattern listed in Table I and by the composition formula (in terms of oxides):

$$0.9 \pm 0.2\ M_{2/n}O : B_2O_3 : ySiO_2 : zH_2O$$

wherein M is at least one cation, n is the valence of the cation, y is between 4 and about 600 and z is between 0 and about 160.

# 0 068 796

TABLE I

| d-Spacing Å (1) | Assigned Strength (2) |
| --- | --- |
| 11.2 ± 0.2 | W—VS |
| 10.0 ± 0.2 | W—MS |
| 5.97 ± 0.07 | W—M |
| 3.82 ± 0.05 | VS |
| 3.70 ± 0.05 | MS |
| 3.62 ± 0.05 | M—MS |
| 2.97 ± 0.02 | W—M |
| 1.99 ± 0.02 | VW—M |

(1) Copper K alpha radiation
(2) VW = very weak; W = weak; M = medium;
   MS = medium strong; VS = very strong

It has been found that preparations of AMS-1B by conventional techniques using sodium hydroxide sometimes contain searlesite as an impurity especially if the concentration of reactants in the crystallizing mixture is high. However, AMS-1B crystalline borosilicate can be prepared according to this invention using higher than conventional concentrations of reactants without producing searlesite. In addition, preparations at higher concentrations of reactants produce a crystalline borosilicate with increased activity in some hydrocarbon conversion processes. Further, higher reactant concentration preparations are economically more efficient.

More specifically, the material of the present invention is prepared by mixing in water (preferably distilled or deionized) ethylenediamine, a boron oxide source, and, optionally, an organic template compound such as tetra-n-propylammonium bromide. The order of addition usually is not critical although a typical procedure is to dissolve ethylenediamine and boric acid in water and then add the template compound. Generally, the silicon oxide compound is added with intensive mixing such as that performed in a Waring Blendor. The resulting slurry is tranferred to a closed crystallization vessel for a suitable time. After crystallization, the resulting crystalline product can be filtered, washed with water, dried, and calcined.

During preparation, acidic conditions should be avoided. Advantageously, the pH of the reaction system falls within the range of about 8 to about 12 and most preferably between about 9 and about 10.5. The pH depends on the concentration of ethylenediamine.

Examples of oxides of silicon useful in this invention include silicic acid, sodium silicate, tetraalkyl silicates and Ludox, a stabilized polymer of silicic acid manufactured by E. I. du Pont de Nemours & Co. Typically, the oxide of boron source is boric acid although equivalent species can be used such as sodium borate and other boron-containing compounds.

Since AMS-1B crystalline borosilicate prepared according to this invention requires no alkali metal cation and thus requires no ion exchange procedure before formulation into a catalytic composition, it is advantageous that the starting materials, such as silicon oxide and boron oxide, contain as little alkali metal ion contaminant as practicable.

Organic templates useful in preparing AMS-1B crystalline borosilicate include alkylammonium cations or precursors thereof such as tetraalkylammonium compounds. Useful organic templates include tetra-n-propylammonium bromide and tetra-n-propylammonium hydroxide.

In a more detailed description of a typical preparation of this invention, suitable quantities of ethylenediamine and boric acid ($H_3BO_3$) are dissolved in distilled or deionized water followed by addition of the organic template. The resulting slurry is transferred to a closed crystallization vessel and reacted usually at a pressure at least the vapor pressure of water for a time sufficient to permit crystallization which usually is about 0.25 to about 20 days, typically is about one to about ten days and preferably is about two to about seven days, at a temperature is maintained below the decomposition temperature ranging from about 100° to about 250°C, preferably about 125° to about 200°C. The crystallizing material can be stirred or agitated as in a rocker bomb. Preferably, the crystallization temperature is maintained below the decomposition temperature of the organic template compound. Especially preferred conditions are crystallizing at about 145°C for about two to about four days. Samples to material can be removed during crystallization to check the degree of crystallization and determine the optimum crystallization time.

4

The crystalline material formed can be separated and recovered by well-known means such as filtration with washing. This material can be mildly dried for anywhere from a few hours to a few days at varying temperatures, typically about 25—200°C, to form a dry cake which can then be crushed to a powder or to small particles and extruded, pelletized, or made into forms suitable for its intended use. Typically, materials prepared after mild drying contain the organic template compound and water of hydration within the solid mass and a subsequent activation or calcination procedure is necessary, if it is desired to remove this material from the final product. Typically, mildly dried product is calcined at temperatures ranging from about 260° to about 850°C and preferably about 525° to about 600°C. Extreme calcination temperatures or prolonged crystallization times may prove detrimental to the crystal structure or may totally destroy it. Generally there is no need to raise the calcination temperature beyond about 600°C in order to remove organic material from the originally formed crystalline material. Typically, the molecular sieve material is dried in a forced draft oven at about 145°—165°C for about 16 hours and is then calcined in air in a manner such that the temperature rise does not exceed 125°C per hour until a temperature of about 540°C is reached. Calcination at this temperature usually is continued for about 4 to 16 hours.

A catalytically active material can be placed onto the borosilicate structure by ion exchange, impregnation, a combination thereof, or other suitable contract means. Preferred replacing cations are those which render the crystalline borosilicate catalytically active, especially for hydrocarbon conversion. Typical catalytically active ions include hydrogen, metal ions of Groups IB, IIA, IIB, IIIA, and VIII, and of manganese, vanadium, chromium, uranium, and rare earth elements.

Also, water soluble salts of catalytically active materials can be impregnated onto the crystalline borosilicate of this invention. Such catalytically active materials include hydrogen, metals of Groups IB, IIA, IIB, IIIA, IVB, VIB, VIIB, and VIII, and rare earth elements.

In another aspect of this invention a catalytically active material can be placed onto the borosilicate structure by incorporating such catalytically active material in the initial crystallization. Generally the same elements can be placed onto the sieve structure in this manner as can be ion exchanged or impregnated. Specific metal ions which can be incorporated in such manner include ions of Ni, Co, Mn, V, Ti, Cu, Zn, Mo an Zr.

Ion exchange and impregnation techniques are well known in the art. Typically, an aqueous solution of a cationic species is exchanged one or more times at about 25° to about 100°C. Impregnation of a catalytically active compound on the borosilicate or on a composition comprising the crystalline borosilicate suspended in and distributed throughout a matrix of a support material such as a porous refractory inorganic oxide such as alumina, often results in a suitable catalytic composition. A combination of ion exchange and impregnation can be used. Presence of sodium ion in a composition usually is detrimental to catalytic activity. AMS-1B-based catalyst compositions useful in xylene isomerization can be based on hydrogen form sieve or on that prepared by ion exchange with nickelous nitrate or by impregnation with ammonium molybdate.

The amount of catalytically active material placed on the AMS-1B borosilicate can vary from less than one weight percent to about thirty weight percent, typically from about 0.05 to about 25 weight percent, depending on the process use intended. The optimum amount can be determined easily by routine experimentation.

The AMS-1B crystalline borosilicate useful in this invention may be incorporated as a pure material in a catalyst or adsorbent, or may be admixed with or incorporated within various binders or matrix materials depending upon the intended process use. The crystalline borosilicate can be combined with active or inactive materials, synthetic or naturally-occurring zeolites, as well as inorganic or organic materials which would be useful for binding the borosilicate. Well-known materials include silica, silica-alumina, alumina, alumina sols, hydrated aluminas, clays such as bentonite or kaoline, or other binders well known in the art. Typically, the borosilicate is incorporated within a matrix material by blending with a sol of the matrix material and gelling the resulting mixture. Also, solid particles of the borosilicate and matrix material can be physically admixed. Typically, such borosilicate compositions can be pelletized or extruded into useful shapes. The crystalline borosilicate content can vary anywhere up to 100 wt.% of the total composition. Catalytic compositions can contain about 0.1 wt.% to about 100 wt.% crystalline borosilicate material and typically contain about 2 wt.% to about 65 wt.% of such material.

Catalytic compositions comprising the crystalline borosilicate material of this invention and a suitable matrix material can be formed by adding a finely-divided crystalline borosilicate and a catalytically active metal compound to an aqueous sol or gel of the matrix material. The resulting mixture is thoroughly blended and gelled typically by adding a material such as aqueous ammonia. The resulting gel can be dried and calcined to form a composition in which the crystalline borosilicate and catalytically active metal compound are distributed throughout the matrix material.

Specific details of catalyst preparations are described in U.S. Patent 4,268,420.

This invention is demonstrated but not limited by the following Examples and Comparative Runs.

Example I

An AMS-1B crystalline borosilicate was prepared using relatively high concentrations of reactants with respect to the water diluent. The molecular sieve was prepared by dissolving ethylenediamine, boric acid, and tetra-n-propylammonium bromide (TPABr) in distilled water. While agitating this mixture in a Waring Blendor at maximum speed, a quantity of Ludox (40 wt.% $SiO_2$) was added quickly; agitation was continued for about 10 minutes. The resulting mixture was charged to a stirred autoclave and digested at 145°C. After the mixture was crystallized, the resulting product was filtered, washed with distilled water, dried overnight at 130°C, and calcined at 530°C for four hours preceded by a programmed preheating at a temperature increase of no more than 125°C/hour. The products were analyzed by x-ray diffraction and elemental analysis. Products characterised as AMS-1B had an x-ray diffraction pattern similar to that contained in Table I and elemental analysis showing incorporation of boron. Details of the preparation and analyses are shown in Table II. A catalyst composition was prepared by dispersing 10 grams of calcined sieve as described above in 405 grams of PHF-alumina hydrosol. The mixture was gelled with 20 milliliters of concentrated aqueous ammonia. The resulting solid was dried over night in a forced air oven at 130°C and then program calcined at 530°C for twelve hours preceded by a temperature increase of 125°C/hour. The calcined solid was crushed and sized to 0.99—0.43 mm (18-40 mesh US Sieve Series) and five grams of such 0.99—0.43 mm (18—40 mesh) catalyst were placed into a micro aromatics test unit having a 1.27 cm (0.5-inch) inside diameter tubular reactor and preconditioned for two hours at 399°C an 1.14 MPa (165 psig) pressure with 0.009 m³ (0.3 SCF) per hour flow of hydrogen. Xylene isomerization test results are shown in Table III.

TABLE II

| Reagents (grams) | EXAMPLE I |
|---|---|
| Water | 5,400 |
| Ethylenediamine | 720 |
| Boric Acid | 920 |
| Tetra-n-propylammonium Bromide | 240 |
| Ludox (HS-40, 40 wt.% $SiO_2$) | 6,000 |
| Mole Ratios of Reagents | |
| $SiO_2/B_2O_3$ | 5.38 |
| $H_2O/SiO_2$ | 12.5 |
| Ethylenediamine/$SiO_2$ | 0.30 |
| TPABr/$SiO_2$ | 0.023 |
| Crystallization conditions | |
| Time (days) | 7 |
| Temperature (°C) | 145 |
| Initial pH | 9.8 |
| Elemental Analysis (wt.%) | |
| B | 1.09 |

# 0 068 796

TABLE III

| Conditions | Test Run for Example I | |
|---|---|---|
| Reactor Temp. (°C) | | 399 |
| Reactor Pressure MPa (psig) | | 1.14 (165) |
| Space Velocity (WHSV, hr$^{-1}$) | | 6.8 |
| Hydrogen/hydrocarbon (molar ratio) | | 4.6 |
| Components (wt.%) | Feed | |
| Paraffins and Naphthenes | 0.00 | 0.02 |
| Benzene | 0.05 | 3.51 |
| Toluene | 0.05 | 0.84 |
| Ethylebenzene | 13.33 | ·7.63 |
| p-Xylene | 10.05 | 19.96 |
| m-Xylene | 53.55 | 43.24 |
| o-Xylene | 22.93 | 19.21 |
| $C_9+$ | 0.06 | 5.60 |
| Results[1] | | |
| PATE — p-Xylene | | 105.8 |
| Ethylbenzene conversion (%) | | 42.7 |

[1] PATE = Percent Approach to Theoretical Equilibrium

Example II and Comparative Runs A and B

A series of preparations of AMS-1B crystalline borosilicate was conducted according to this invention to show the substantial increase hydrocarbon conversion catalytic activity of AMS-1B material made using increased concentrations of reactants with respect to water. The AMS-1B crystalline borosilicate of Example II was prepared using ethylenediamine with no added metal hydroxide and with a low water to silica molar ratio. The material prepared in Example II is similar to that prepared in Example I. The AMS-1B of Run A was prepared in a manner similar to that described in Example I using a higher water to silica molar ratio. Comparative Run B was prepared using sodium hydroxide as the base with no ethylenediamine.

Xylene isomerization/ethylbenzene conversion tests using catalysts prepared from the materials of Example II and Comparative Runs A and B show the catalyst prepared from the Example II material to have a substantially higher ethylbenzene conversion activity as compared to similarly-formulated catalysts made from the other materials.

The AMS-1B crystalline borosilicate molecular sieve of Example II was prepared by mixing in an autoclave distilled water, ethylenediamine, boric acid, tetra-n-propylammonium bromide and Ludox HS-40 silica sol (40 wt.% solids). The resulting mixture was digested for four days at 145°C, after which time the product was washed thoroughly with distilled water, dried at 130°C for 16 hours and calcined at 535°C for 12 hours after a programmed rate of heating of 125°C/hour for four hours. The resulting molecular sieve had particle sizes of 0.1—0.5 micrometers. Mole ratios of reagents were $SiO_2/B_2O_3 = 5.38$; $H_2O/SiO_2 = 15$; ethylenediamine/$SiO_2 = 0.30$; TPABr/$SiO_2 = 0.023$. The AMS-1B crystalline borosilicate of Example II had a boron content of 0.85 wt.%.

Catalyst compositions were prepared by dispersing the above-prepared sieve in 1667 grams of PHF gamma alumina hydrosol (9.6 wt.% solids) and gelling with 80 milliliters of concentrated aqueous ammonia (28 wt.% $NH_3$). Several catalysts were prepared using different sieve/alumina matrix weight

7

ratios. The following amounts of sieve were used for the corresponding sieve/alumina matrix weight ratios: 20/80 = 40.0 grams; 30/70 = 68.6 grams; 35/65 = 86.2 grams; 40/60 =106.7 grams; 45/55 = 130.9 grams; and 55/45 = 195.6 grams. The gelled solid was dried overnight in a forced air oven at 130°C, ground to 0.99—0.43 mm (18—40 mesh U.S. Sieve Series), and then calcined at 537°C for 12 hours preceded by a temperature increase of 125°C/hour. Five to ten grams of the resulting calcined catalyst was placed into a micro aromatics test unit having a 1.27 cm (0.5-inch) inside diameter tubular reactor and preconditioned for two hours at 371°C and 1.74 MPa (250 psig) pressure with 0.009 $m^3$ (0.3 SCF) per hour hydrogen flow. Xylene isomerization/ethylbenzene conversion test results are shown in Table IV.

AMS-1B crystalline borosilicate molecular sieve of Run A was prepared in a manner similar to that described in Example I using Ludox HS-40 (40 wt.% $SiO_2$), tetrapropylammonium bromide, boric acid, ethylenediamine and water such that the molar ratios of reactants were $SiO_2/B_2O_3$ = 5.38; $H_2O/SiO_2$ = 30; ethylenediamine/$SiO_2$ = 0.30 and TPABr/$SiO_2$ = 0.023. The reactant mixture was digested at 132—136°C for 4.5 days after which time the resulting solids were washed thoroughly with distilled water, dried at 130°C and calcined at 530°C. The resulting AMS-1B crystalline borosilicate molecular sieve had particle sizes of 0.2—2 micrometers and a boron content of 0.85 wt.%. Catalyst compositions with various sieve/alumina matrix weight ratios were prepared as described for Example II.

For a 20/80 sieve/alumina matrix catalyst, 417 grams of PHF gamma alumina sol (9.8 wt.% solids), 10.0 grams of sieve and 60 milliliters of concentrated aqueous ammonia (28 wt.% $NH_3$) gelling agent were used; for a 30/70 silica/matrix catalyst, 1215.9 grams of alumina sol, 51.39 grams of sieve and 120 milliliters of aqueous ammonia were used; for a 35/65 catalyst 1215.9 grams of alumina sol, 64.56 grams of sieve and 60 milliliters of aqueous ammonia were used; and for a 40/60 catalyst 405.3 grams of alumina sol, 26.67 grams of sieve and 60 milliliters of aqueous ammonia were used. These catalyst compositions were tested for xylene isomerization/ethylbenzene conversion as described for Example II and the results shown in Table V.

AMS-1B crystalline borosilicate molecular sieve of Comparative Run B was prepared by digesting a mixture of water, boric acid, sodium hydroxide, tetrapropylammonium bromide and Ludox HS-40 (40 wt.% $SiO_2$) for 2.5 days at 145°C. The molar ratios of reactants were: $SiO_2/B_2O_3$ = 5.06; $H_2O/SiO_2$ = 30.5; NaOH/$SiO_2$ = 0.42; and TPABr/$SiO_2$ = 0.14. Resulting solids were washed with water, dried and calcined. The calcined sieve then was exchanged twice with an ammonium acetate solution at 90°C for two hours. Two grams of ammonium acetate in ten grams of water per gram of sieve were used in the exchanges. The resulting exchanged sieve was dried and calcined and then formulated into catalysts incorporated into a gamma alumina matrix as described above for Example II. The AMS-1B crystalline borosilicate of Run B had particle sizes of 0.1—0.5 micrometers and a boron content of 0.5 wt.%. The quantities of PHF alumina sol (9.7 wt.% solids), sieve and aqueous ammonia for various sieve/alumina matrix weight ratios are: 20/80—2060 grams alumina sol, 50 grams of sieve and 400 milliliters aqueous ammonia; 30/70—1500 grams alumina sol, 62.3 grams of sieve and 218 milliliters aqueous ammonia; 35/65—1675.3 grams alumina sol, 87.5 grams of sieve and 325 milliliters of aqueous ammonia; 40/60—1546.4 grams alumina sol, 100 grams of sieve and 300 milliliters of aqueous ammonia. These catalyst compositions were tested for xylene isomerization/ethylbenzene conversion as described for Example II and the results are shown in Table VI.

The data show that catalytic materials formulated from AMS-1B crystalline borosilicate molecular sieve of Example II are significantly more active for ethylbenzene conversion than similarly formulated materials prepared as in Runs A and B.

TABLE IV

| Sieve/Alumina | Test Runs from Example IV | | | |
|---|---|---|---|---|
| Matrix (wt. ratio) | 20/80 | | 30/70 | |
| Conditions | | | | |
| Reactor Temp. (°C) | 372 | | 371 | |
| Reactor Pressure MPa (psig) | 1.74 (250) | | 1.74 (250) | |
| Space Velocity (WHSV, hr$^{-1}$) | 6.0 | | 6.1 | |
| Hydrogen/hydrocarbon (molar ratio) | 2.1 | | 1.9 | |
| Components | | | | |
| (wt.%) | Feed | | Feed | |
| Paraffins and Naphthenes | 0.01 | 0.08 | 0 | 0.02 |
| Benzene | 0.04 | 2.03 | 0.05 | 2.43 |
| Toluene | 0.05 | 0.56 | 0.05 | 0.80 |
| Ethylbenzene | 13.90 | 10.35 | 13.33 | 8.89 |
| p-Xylene | 10.32 | 20.22 | 10.05 | 20.11 |
| m-Xylene | 52.90 | 44.06 | 53.55 | 43.85 |
| o-Xylene | 22.71 | 19.03 | 22.93 | 19.05 |
| $C_{9\div}$ | 0.07 | 3.67 | 0.05 | 4.85 |
| Results[1] | | | | |
| PATE — | | | | |
| p-Xylene | | 105.6 | | 105.0 |
| Ethylbenzene conversion (%) | | 25.6 | | 33.3 |
| Xylene Loss (wt.%) | | 3.06 | | 4.27 |

[1] PATE = Percent Approach to Theoretical Equilibrium

TABLE IV (continued)

Test Runs from Example IV

| Sieve/Alumina | | | | |
|---|---|---|---|---|
| Matrix (wt. ratio) | 35/65 | | 40/60 | |
| **Conditions** | | | | |
| Reactor Temp. (°C) | 371 | | 371 | |
| Reactor Pressure MPa (psig) | 1.74 (250) | | 1.74 (250) | |
| Space Velocity (WHSV, hr$^{-1}$) | 6.0 | | 6.0 | |
| Hydrogen/hydrocarbon (molar ratio) | 2.0 | | 2.0 | |
| Components | | | | |
| (wt.%) | Feed | | Feed | |
| Paraffins and Naphthenes | 0.01 | 0.14 | 0.01 | 0.19 |
| Benzene | 0.04 | 3.39 | 0.04 | 3.94 |
| Toluene | 0.05 | 0.96 | 0.05 | 1.21 |
| Ethylbenzene | 13.90 | 8.24 | 13.90 | 7.28 |
| p-Xylene | 10.32 | 19.67 | 10.32 | 19.36 |
| m-Xylene | 52.90 | 42.94 | 52.90 | 42.58 |
| o-Xylene | 22.71 | 18.61 | 22.71 | 18.25 |
| $C_{9\div}$ | 0.07 | 6.05 | 0.07 | 7.19 |
| Results[1] | | | | |
| PATE — | | | | |
| p-Xylene | | 105.1 | | 104.5 |
| Ethylbenzene conversion (%) | | 40.7 | | 47.6 |
| Xylene Loss (wt.%) | | 5.43 | | 6.72 |

[1] PATE = Percent Approach to Theoretical Equilibrium

TABLE IV (continued)

Test Runs from Example IV

| Sieve/Alumina Matrix (wt. ratio) | | 45/55 | | 55/45 |
|---|---|---|---|---|
| **Conditions** | | | | |
| Reactor Temp. (°C) | | 371 | | 372 |
| Reactor Pressure MPa (psig) | | 1.74 (250) | | 1.74 (250) |
| Space Velocity (WHSV, hr⁻¹) | | 6.0 | | 6.0 |
| Hydrogen/hydrocarbon (molar ratio) | | 2.0 | | 2.0 |
| **Components** | | | | |
| (wt.%) | Feed | | Feed | |
| Paraffins and Naphthenes | 0 | 0.03 | 0.01 | 0.16 |
| Benzene | 0.05 | 3.14 | 0.04 | 3.08 |
| Toluene | 0.05 | 1.07 | 0.05 | 0.81 |
| Ethylbenzene | 13.33 | 7.68 | 13.90 | 8.67 |
| p-Xylene | 10.05 | 19.79 | 10.32 | 19.92 |
| m-Xylene | 53.55 | 43.32 | 52.90 | 43.36 |
| o-Xylene | 22.93 | 18.74 | 22.71 | 18.83 |
| C₉₋ | 0.05 | 6.23 | 0.07 | 5.17 |
| **Results[1]** | | | | |
| PATE — | | | | |
| p-Xylene | | 104.5 | | 105.5 |
| Ethylbenzene conversion (%) | | 42.5 | | 37.7 |
| Xylene Loss (wt.%) | | 5.76 | | 4.48 |

[1] PATE = Percent Approach to Theoretical Equilibrium

11

TABLE V

| | Test Runs from Run A | | | |
|---|---|---|---|---|
| Sieve/Alumina Matrix (wt. ratio) | 35/65 | | 40/60 | |
| **Conditions** | | | | |
| Reactor Temp. (°C) | 372 | | 371 | |
| Reactor Pressure MPa (psig) | 1.74 (250) | | 1.74 (250) | |
| Space Velocity (WHSV, $hr^{-1}$) | 6.0 | | 5.9 | |
| Hydrogen/hydrocarbon (molar ratio) | 2.0 | 2.1 | | |
| **Components** (wt.%). | Feed | | Feed | |
| Paraffins and Naphthenes | 0.01 | 0.09 | 0 | 0.02 |
| Benzene | 0.04 | 2.77 | 0.04 | 1.88 |
| Toluene | 0.05 | 0.80 | 0.06 | 0.77 |
| Ethylbenzene | 13.90 | 9.12 | 14.01 | 10.51 |
| p-Xylene | 10.32 | 20.00 | 10.36 | 20.09 |
| m-Xylene | 52.90 | 43.49 | 52.73 | 44.00 |
| o-Xylene | 22.71 | 18.88 | 22.74 | 18.89 |
| $C_{9+}$ | 0.07 | 4.86 | 0.06 | 3.85 |
| **Results[1]** | | | | |
| PATE — | | | | |
| p-Xylene | 105.6 | | 105.1 | |
| Ethylbenzene conversion (%) | 34.4 | | 25.0 | |
| Xylene Loss (wt.%) | 4.16 | | 3.41 | |

[1] PATE = Percent Approach to Theoretical Equilibrium

TABLE V (continued)

| Sieve/Alumina | Test Runs from Run A | | | |
|---|---|---|---|---|
| Matrix (wt. ratio) | | 20/80 | | 30/70 |
| **Conditions** | | | | |
| Reactor Temp. (°C) | | 372 | | 372 |
| Reactor Pressure MPa (psig) | | 1.74 (250) | | 1.74 (250) |
| Space Velocity (WHSV, hr$^{-1}$) | | 6.1 | | 6.3 |
| Hydrogen/hydrocarbon (molar ratio) | | 2.2 | | 1.9 |
| Components | | | | |
| (wt.%) | Feed | | Feed | |
| Paraffins and Naphthenes | 0 | 0.02 | 0 | 0.03 |
| Benzene | 0.05 | 1.67 | 0.05 | 2.23 |
| Toluene | 0.05 | 0.59 | 0.05 | 0.95 |
| Ethylbenzene | 13.33 | 10.19 | 13.33 | 9.34 |
| p-Xylene | 10.05 | 20.42 | 10.05 | 20.09 |
| m-Xylene | 53.55 | 44.53 | 53.55 | 43.68 |
| o-Xylene | 22.93 | 19.31 | 22.93 | 19.02 |
| C$_{9+}$ | 0.05 | 3.28 | 0.06 | 4.66 |
| Results[1] | | | | |
| PATE — | | | | |
| p-Xylene | | 105.1 | | 105.3 |
| Ethylbenzene conversion (%) | | 23.6 | | 29.9 |
| Xylene Loss (wt.%) | | 2.81 | | 4.31 |

[1] PATE = Percent Approach to Theoretical Equilibrium

**0 068 796**

TABLE VI

Test Runs from Run B

| Sieve/Alumina | | | | |
|---|---|---|---|---|
| Matrix (wt. ratio) | | 35/65 | | 40/60 |
| Conditions | | | | |
| Reactor Temp. (°C) | | 373 | | 373 |
| Reactor Pressure MPa (psig) | | 1.74 (250) | | 1.74 (250) |
| Space Velocity (WHSV, $hr^{-1}$) | | 6.0 | | 6.0 |
| Hydrogen/hydrocarbon (molar ratio) | | 2.0 | | 2.0 |
| Components | | | | |
| (wt.%) | Feed | | Feed | |
| Paraffins and Naphthenes | 0 | 0.01 | 0 | 0.07 |
| Benzene | 0.03 | 2.37 | 0.03 | 2.55 |
| Toluene | 0.05 | 0.58 | 0.05 | 0.62 |
| Ethylbenzene | 14.32 | 10.22 | 14.32 | 10.00 |
| p-Xylene | 10.14 | 19.94 | 10.14 | 19.86 |
| m-Xylene | 52.24 | 43.55 | 52.24 | 43.29 |
| o-Xylene | 23.18 | 18.94 | 23.18 | 18.88 |
| $C_{9+}$ | 0.04 | 4.41 | 0.04 | 4.74 |
| Results[1] | | | | |
| PATE — | | | | |
| p-Xylene | | 104.8 | | 105.0 |
| Ethylbenzene conversion (%) | | 28.6 | | 30.2 |
| Xylene Loss (wt.%) | | 3.49 | | 3.84 |

[1] PATE = Percent Approach to Theoretical Equilibrium

TABLE VI (continued)

Test Runs from Run B

| Sieve/Alumina Matrix (wt. ratio) | 20/80 | | 30/70 | |
|---|---|---|---|---|
| **Conditions** | | | | |
| Reactor Temp. (°C) | 373 | | 371 | |
| Reactor Pressure MPa (psig) | 1.74 (250) | | 1.74 (250) | |
| Space Velocity (WHSV, hr$^{-1}$) | 6.0 | | 6.0 | |
| Hydrogen/hydrocarbon (molar ratio) | 2.0 | | 1.8 | |
| **Components (wt.%)** | Feed | | Feed | |
| Paraffins and Naphthenes | 0 | 0 | 0.16 | 1.15 |
| Benzene | 0.03 | 1.33 | 0 | 2.27 |
| Toluene | 0.05 | 0.32 | 0.85 | 1.68 |
| Ethylbenzene | 14.32 | 12.17 | 14.65 | 10.97 |
| p-Xylene | 10.14 | 20.41 | 7.79 | 18.30 |
| m-Xylene | 52.24 | 44.08 | 49.74 | 40.00 |
| o-Xylene | 23.18 | 19.24 | 21.13 | 16.90 |
| C$_{9+}$ | 0.04 | 2.45 | 4.67 | 8.73 |
| **Results[1]** | | | | |
| PATE — | | | | |
| p-Xylene | 106.3 | | 105.2 | |
| Ethylbenzene conversion (%) | 15.0 | | 25.1 | |
| Xylene Loss (wt.%) | 1.84 | | 4.51 | |

[1] PATE = Percent Approach to Theoretical Equilibrium

**Claims**

1. A method of preparing AMS-1B crystalline borosilicate molecular sieve comprising reacting under crystalline conditions, in substantial absence of a metal or ammonium hydroxide, an aqueous mixture containing an oxide of silicon, an oxide of boron, ethylenediamine and, optionally, an alkylammonium cation or precursor of an alkylammonium cation wherein the molar ratio of silica to oxide of boron is from 2 to 400 and the molar ratio of ethylene diamine to oxide of silicon is above 0.05, characterised in that the molar ratio of water to silica is from 10 to 15.

2. A method according to Claim 1 wherein the molar ratio of alkylammonium cation or precursor of an alkylammonium cation to silica is between 0.005 and 1.0.

3. A method according to Claim 1 or Claim 2 wherein the alkylammonium cation is tetra-n-propylammonium cation.

4. A method according to Claim 3 wherein the molar ratio of tetra-n-propylammonium cation or precursor to silica is 0.01 to 0.1, the molar ratio of ethylenediamine to silica is 0.1 to 1.0 and the molar ratio of silica to oxide of boron is 5 to 80.

5. A method according to Claim 4 wherein the molar ratio of ethylenediamine to silica is 0.2 to 0.5 and the molar ratio of tetra-n-propylammonium cation or precursor to silica is 0.02 to 0.05.

6. A method according to any preceding claim wherein the source for oxide of boron is boric acid.

7. A method according to any preceding claim wherein the crystallizing mixture is maintained at 125°C to 200°C for one to ten days.

8. A method according to any preceding claim wherein a catalytically active material is placed on the borosilicate.

9. A method according to any preceding claim wherein the molecular sieve is incorporated within a suitable matrix material.

10. A method according to Claim 9 wherein the matrix material is silica, silica-alumina or alumina.

11. A method according to any preceding claim wherein ions of nickel, cobalt, manganese, vanadium, titanium, copper, zinc, molybdenum or zirconium are incorporated within the crystallizing mixture.

12. A method of preparing AMS-1B crystalline borosilicate molecular sieve comprising reacting under crystallization conditions, in substantial absence of a metal or ammonium hydroxide, an aqueous mixture containing an oxide of silicon an oxide of boron, ethylenediamine in a molar ratio to silica of above 0.05, and, optionally, an alkylammonium cation or precursor of an alkylammonium cation; wherein the molar ratio of silica to oxide of boron is 4 to 150 and characterised in that the molar ratio of water to silica is 10 to 15.

13. A method according to Claim 12 wherein the molar ratio of alkylammonium cation or precursor to silica is 0.01 to 0.1 and the molar ratio of ethylenediamine to silica is 0.1 to 1.0.

14. A method according to Claim 12 or 13 wherein the alkylammonium cation is tetra-n-propylammonium cation.

15. A method according to Claim 14 wherein the molar ratio of tetra-n-propylammonium cation to silica is 0.01 to 0.1, the molar ratio of silica to oxide of boron is 5 to 80, and the molar ratio of ethylenediamine to silica is 0.01 to 1.0.

16. A method according to Claim 15 wherein the molar ratio of tetra-n-propylammonium cation to silica is 0.02 to 0.05, the molar ratio of silica to oxide of boron is 5 to 20 and the molar ratio of ethylenediamine to silica is 0.2 to 0.5.

17. A method according to Claim 14, 15 or 16 wherein the source for tetra-n-propylammonium cation is tetra-n-propylammonium bromide.

**Patentansprüche**

1. Verfahren zur Herstellung eines Molekularsiebes aus kristallinem AMS-1B Borsilicat, indem man in praktischer Abwesenheit eines Metallhydroxids oder eines Ammoniumhydroxids unter Kristallisationsbedingungen ein wäßriges Gemisch, das ein Siliciumdioxid, ein Boroxid, Ethylendiamin und gegebenenfalls ein Alkylammoniumkation oder einen Vorläufer für ein Alkylammoniumkation enthält, worin das Molverhältnis von Siliciumdioxid zu Boroxid von 2 bis 400 reicht und das Molverhältnis von Ethylendiamin zu Siliciumdioxid über 0,05 ausmacht, miteinander umsetzt, dadurch gekennzeichnet, daß man bei einem Molverhältnis von Wasser zu Siliciumdioxid von 10 bis 15 arbeitet.

2. Verfahren nach Anspruch 1, dadurch gekennzeichnet, daß man bei einem Molverhältnis von Alkylammoniumkation oder Vorläufer für ein Alkylammoniumkation zu Siliciumdioxid von 0,005 bis 1,0 arbeitet.

3. Verfahren nach Anspruch 1 oder 2, dadurch gekennzeichnet, daß man als Alkylammoniumkation ein Tetra-n-propylammoniumkation verwendet.

4. Verfahren nach Anspruch 3, dadurch gekennzeichnet, daß man bei einem Molverhältnis von Tetra-n-propylammoniumkation oder einem Vorläufer hierfür zu Siliciumdioxid von 0,01 bis 0,1, einem Molverhältnis von Ethylendiamin zu Siliciumdioxid von 0,1 bis 1,0 und einem Molverhältnis von Siliciumdioxid zu Boroxid von 5 bis 80 arbeitet.

5. Verfahren nach Anspruch 4, dadurch gekennzeichnet, daß man bei einem Molverhältnis von Ethylendiamin zu Siliciumdioxid von 0,2 bis 0,5 und einem Molverhältnis von Tetra-n-propylammoniumkation oder Vorläufer hierfür zu Siliciumdioxid von 0,02 bis 0,05 arbeitet.

6. Verfahren nach irgendeinem der vorhergehenden Ansprüche, dadurch gekennzeichnet, daß man als Quelle für Boroxid Borsäure verwendet.

7. Verfahren nach irgendeinem der vorhergehenden Ansprüche, dadurch gekennzeichnet, daß man das kristallisierende Gemisch 1 bis 10 Tage auf 125 bis 200°C hält.

8. Verfahren nach irgendeinem der vorhergehenden Ansprüche, dadurch gekennzeichnet, daß man auf das Borsilicat ein katalytisch aktives Material aufbringt.

9. Verfahren nach irgendeinem der vorhergehenden Ansprüche, dadurch gekennzeichnet, daß man das Molekularsieb in ein geeignetes Matrixmaterial einarbeitet.

10. Verfahren nach Anspruch 9, dadurch gekennzeichnet, daß man als Matrixmaterial Siliciumdioxid, Siliciumdioxid-Aluminiumoxid oder Aluminiumoxid verwendet.

11. Verfahren nach irgendeinem der vorhergehenden Ansprüche, dadurch gekennzeichnet, daß man das kristallisierende Gemisch mit Ionen von Nickel, Kobalt, Mangan, Vanadium, Titan, Kupfer, Zink, Molybdän oder Zirkonium versetzt.

12. Verfahren zur Herstellung eines Molekularsiebes aus kristallinum AMS-1B Borsilicat, indem man in praktischer Abwesenheit eines Metallhydroxids oder eines Ammoniumhydroxids unter Kristallisationsbedingungen ein wäßriges Gemisch, das ein Siliciumdioxid, ein Boroxid, Ethylendiamin in einem Molverhältnis zu Siliciumdioxid von über 0,05 und gegebenenfalls ein Alkylammoniumkation oder einen Vorläufer für ein Alkylammoniumkation enthält, worin das Molverhältnis von Siliciumdioxid zu Boroxid von 4 bis 150 reicht, miteinander umsetzt, dadurch gekennzeichnet, daß man bei einem Molverhältnis von Wasser zu Siliciumdioxid von 10 bis 15 arbeitet.

13. Verfahren nach Anspruch 12, dadurch gekennzeichnet, daß man bei einem Molverhältnis von Alkylammoniumkation oder Vorläufer hierfür zu Siliciumdioxid von 0,01 bis 0,1 und unter einem Molverhältnis von Ethylendiamin zu Siliciumdioxid von 0,1 bis 1,0 arbeitet.

14. Verfahren nach Anspruch 12 oder 13, dadurch gekennzeichnet, daß man als Alkylammoniumkation ein Tetra-n-propylammoniumkation verwendet.

15. Verfahren nach Anspruch 14, dadurch gekennzeichnet, daß man unter einem Molverhältnis von Tetra-n-propylammoniumkation zu Siliciumdioxid von 0,01 bis 0,1, einem Molverhältnis von Siliciumdioxid zu Boroxid von 5 bis 80 und einem Molverhältnis von Ethylendiamin zu Siliciumdioxid von 0,1 bis 1,0 arbeitet.

16. Verfahren nach Anspruch 15, dadurch gekennzeichnet, daß man unter einem Molverhältnis von Tetra-n-propylammoniumkation zu Siliciumdioxid von 0,02 bis 0,05, einem Molverhältnis von Siliciumdioxid zu Boroxid von 5 bis 20 und einem Molverhältnis von Ethylendiamin zu Siliciumdioxid von 0,2 bis 0,5 arbeitet.

17. Verfahren nach Anspruch 14, 15 oder 16, dadurch gekennzeichnet, daß man als Quelle für ein Tetra-n-propylammoniumkation Tetra-n-propylammoniumbromid verwendet.

## Revendications

1. Un procédé de préparation d'un tamis moléculaire en borosilicate cristallin AMS-1B comprenant la réaction dans des conditions de cristallisation, essentiellement en l'absence d'un hydroxyde de métal ou d'ammonium, d'un mélange aqueux contenant un oxyde de silicium, un oxyde de bore, de l'éthylènediamine et éventuellement un cation alcoylammonium ou un précurseur d'un cation alcoylammonium dans lequel le rapport molaire de la silice à l'oxyde de bore est de 2 à 400 et le rapport molaire de l'éthylènediamine à l'oxyde de silicium est supérieur à 0,05, caractérisé en ce que le rapport molaire de l'eau à la silice est de 10 à 15.

2. Un procédé selon la revendication 1 dans lequel le rapport molaire du cation alcoylammonium ou du précurseur d'un cation alcoylammonium à la silice est entre 0,005 et 1,0.

3. Un procédé selon la revendication 1 ou la revendication 2 dans lequel le cation alcoylammonium est le cation tétra-n-propylammonium.

4. Un procédé selon la revendication 3 dans lequel le rapport molaire du cation tétra-n-propylammonium ou de son précurseur à la silice est de 0,01 à 0,1, le rapport molaire de l'éthylènediamine à la silice est de 0,1 à 1,0 et le rapport molaire de la silice à l'oxyde de bore est de 5 à 80.

5. Un procédé selon la revendication 4 dans lequel le rapport molaire de l'éthylènediamine à la silice est de 0,2 à 0,5 et le rapport molaire du cation tétra-n-propylammonium ou de son précurseur à la silice est de 0,02 à 0,05.

6. Un procédé selon l'une quelconque des revendications précédentes dans lequel la source d'oxyde de bore est l'acide borique.

7. Un procédé selon l'une quelconque des revendications précédentes dans lequel le mélange cristallisant est maintenu entre 125° et 200°C pendant 1 à 10 jours.

8. Un procédé selon l'une quelconque des revendications précédentes dans lequel une matiére catalytiquement active est placée sur le borosilicate.

9. Un procédé selon l'une quelconque des revendications précédentes dans lequel le tamis moléculaire est incorporé à une matrice appropriée.

10. Un procédé selon la revendication 9 dans lequel la matrice est la silice, la silice-alumine ou l'alumine.

11. Un procédé selon l'une quelconque des revendications précédentes dans lequel des ions de nickel, de cobalt, de manganèse, de vanadium, de titane, de cuivre, de zinc, de molybdène ou de zirconium sont incorporés au mélange cristallisant.

12. Un procédé de préparation d'un tamis moléculaire en borosilicate cristallin AMS-1B comprenant la réaction dans des conditions de cristallisation, essentiellement en l'absence d'hydroxyde de métal ou d'ammonium, d'un mélange aqueux contenant un oxyde de silicium, un oxyde de bore, et de l'éthylènediamine dans un rapport molaire à la silice supérieur à 0,05 et éventuellement un cation alcoylammonium ou un précurseur d'un cation alcoylammonium; dans lequel le rapport molaire de la silice à l'oxyde de bore est de 4 à 150 et caractérisé en ce que le rapport molaire de l'eau à la silice est de 10 à 15.

13. Un procédé selon la revendication 12 dans lequel le rapport molaire du cation alcoylammonium ou de son précurseur à la silice est de 0,01 à 0,1 et le rapport molaire de l'éthylènediamine à la silice est de 0,1 à 1,0.

14. Un procédé selon la revendication 12 ou 13 dans lequel le cation alcoylammonium est le cation tétra-n-propylammonium.

15. Un procédé selon la revendication 14 dans lequel le rapport molaire du cation tétra-n-propylammonium à la silice est de 0,01 à 0,1, le rapport molaire de la silice à l'oxyde de bore est de 5 à 80 et le rapport molaire de l'éthylènediamine à la silice est de 0,1 à 1,0.

16. Un procédé selon la revendication 15 dans lequel le rapport molaire du cation tétra-n-propylammonium à la silice est de 0,02 à 0,05, le rapport molaire de la silice à l'oxyde de bore est 5 à 20 et le rapport molaire de l'éthylènediamine à la silice est de 0,2 à 0,5.

17. Un procédé selon la revendication 14, 15 ou 16 dans lequel la source de cation tétra-n-propylammonium est le bromure de tétra-n-propylammonium.